# FASCICULE DE BREVET EUROPEEN

(11) **EP 3 175 825 B1**
(45) Date de publication et mention de la délivrance du brevet: **08.08.2018**
(21) Numéro de dépôt: 16198792.0
(22) Date de dépôt: 15.11.2016
(51) Int. Cl.: A61F 2/38

(54) **PARTIE FEMORALE D'UNE PROTHESE DU GENOU DITE POSTERO STABILISEE**
FEMORTEIL EINER SOGENANNTEN POSTERIOR STABILISIERTEN KNIEPROTHESE (PS-KNIE)
FEMORAL PORTION OF A SO-CALLED REAR-STABILISED KNEE PROSTHESIS

(30) Priorité: 02.12.2015 FR 1502514
(43) Date de publication de la demande: 07.06.2017
(73) Titulaire: XNOV IP, 1882 Luxembourg (LU)
(72) Inventeur: MERTI, Patrice, 80000 Amiens (FR); GABRION, Antoine, 80000 Amiens (FR); MARCHETTI, Emmanuel, 38300 Bourgoin Jallieu (FR); CHALENCON, François, 42100 Saint Etienne (FR); FAYARD, Jean Marie, 69390 Vourles (FR); RADWAN, Hilmi, 69620 Bagnols (FR); FORNASIERI, Christophe, 38240 Meylan (FR); PINOIT, Yannick, 59000 Lille (FR); MOREAU, Pascal, 10000 Troyes (FR); BRONNER, Joseph, 68000 Mulhouse (FR); MULLIEZ, Arnaud, 2904 Bressaucourt (CH); LOEHLE, Pascal, 2900 Porrentruy (CH); BIEGUN, Frédérique, 2900 Porrentruy (CH); BIEGUN, Jean-François, 2900 Porrentruy (CH); MARCEAUX, Pascal, 52000 Chaumont (FR)
(74) Mandataire: Eidelsberg, Olivier Nathan

(56) Documents cités:
- EP-A1- 0 123 016
- DE-A1- 3 334 531
- FR-A1- 2 959 410
- US-A1- 2013 190 884

## Description

La présente invention se rapporte à une prothèse du genou dite postéro stabilisée. Classiquement, une telle prothèse comporte une partie fémorale destinée à être fixée au fémur, notamment par ancrage, et une partie tibiale destinée à être fixée au tibia, notamment également par ancrage. Entre la partie tibiale et la partie fémorale, il est prévu un insert, ou ménisque, qui peut être fixé à la partie tibiale ou mobile par rapport à celle ci, notamment en un matériau plus tendre que celui en lequel sont réalisées les parties fémorale et tibiale, par exemple en polyéthylène. Dans sa partie supérieure, l'insert en polyéthylène comporte des surfaces généralement concaves, dans lesquelles viennent en contact de glissement et/ou de roulement les surfaces extérieures de deux condyles de la partie fémorale. Un plot tibial, fait saillie de la face supérieure de la base de l'insert, en particulier perpendiculairement, et pénètre dans une ouverture formée entre les deux condyles de la partie fémorale. La partie fémorale comporte, du côté postérieur, une came fémorale, qui s'étend d'un condyle à l'autre. Il est ainsi formé dans la partie fémorale, délimitée par les deux condyles d'une part et la came fémorale d'autre part, un espace que l'on appelle cage de postéro stabilisation, dans lequel est reçu le plot tibial lors de la coopération des deux parties fémorale et tibiale pour former la prothèse totale de genou.

En raison de l'équilibrage des ligaments latéraux, ces prothèses, comme d'ailleurs toutes les prothèses de genou, sont soumises à un phénomène de bascule dans le plan frontal, classiquement appelé effet « lift-off ». Cet effet « lift-off » consiste en un basculement de la partie fémorale par rapport à la partie tibiale suivant un axe de basculement s'étendant suivant la direction antéropostérieure. L'un des condyles presse plus fort contre l'insert en polyéthylène, tandis que l'autre condyle se soulève. Il en résulte une usure prématurée du plot du ménisque.

La présente invention vise à surmonter les inconvénients de l'art antérieur par une prothèse de genou postéro stabilisée du genre ci-dessus, qui supporte mieux l'effet « lift-off » et notamment qui évite une usure prématurée du plot en polyéthylène du ménisque, ce qui évite d'avoir à le changer, et donc notamment évite d'avoir à réopérer le patient pour remplacer son ménisque usé prématurément ou palier d'autres complications liées à l'usure du PE.

Aux documents FR 2 959 410 et US 2013/190884, il est décrit une partie fémorale suivant le préambule de la revendication 1.

Suivant l'invention, une prothèse totale de genou est telle que définie à la revendication 1, des perfectionnements et mode de réalisation avantageux ou préférés étant définis aux sous revendications. Suivant l'invention, les flancs gauche et droit de la cage sont inclinés respectivement par rapport aux faces gauche et droite du plot, notamment d'un angle d'environ 5°.

Suivant l'invention, les faces opposées du plot sont verticales, tandis que les faces d'au moins l'une, de préférence des deux flancs de la cage, sont inclinées en s'évasant vers l'extérieur.

Suivant l'invention, la forme des flancs de la cage est réalisée de sorte que, pour tout angle de flexion de la prothèse, notamment compris entre 0 et 135°, le flanc est incliné par rapport aux faces latérales opposées du plot l'insert.

Suivant l'invention, l'angle d'inclinaison augmente au fur à mesure que l'on se déplace dans la direction antéro-postérieure, de l'avant vers l'arrière, c'est à dire au fur et à mesure que la flexion augmente.

De préférence, les faces gauche et droite opposées du plot sont planes, de préférence mutuellement parallèles.

En prévoyant ainsi des flancs inclinés de manière à ce que la cage de postéro stabilisation soit de forme conique, on s'assure que, lors d'un basculement lié au fait que l'implant se soulève latéralement (effet lift-off), il est réalisé du côté du condyle non soulevé, un contact plan (ou, en section, linéaire) entre la face du plot de la came et le flanc du condyle, et non plus comme suivant l'art antérieur un contact linéaire (ou, en section, ponctuel) qui use le matériau le plus tendre, par exemple le polyéthylène. Le contact surface sur surface assure une moindre dégradation du polyéthylène.

La présente invention se rapporte également à une prothèse totale du genou comportant une partie tibiale, un ménisque et une partie fémorale suivant l'invention.

A titre d'exemple, on décrit maintenant un mode de réalisation préféré de l'invention en se reportant aux dessins dans lesquels :
- la figure 1: représente suivant une vue en perspective une partie fémorale d'une prothèse de genou postéro stabilisée suivant l'invention ;
- la figure 2: représente suivant une vue en coupe dans un plan perpendiculaire à la direction antéro postérieure des positions relatives de la partie fémorale et de l'insert tibial dans le cas d'un basculement ou lift-off, pour une position de flexion d'environ 60° ;
- la figure 3: est une vue en coupe dans le plan saggital de la partie fémorale de la figure 1 ;
- les figures 4A, 4B et 4C: sont des vues en coupe respectivement suivant les lignes A-A, B-B et C-C de la figure 3 ; et
- la figure 5: représente la courbe donnant la variation de l'angle d'inclinaison en fonction de la flexion pour la prothèse de la figure 3.

Aux figures, il est représentée une prothèse totale du genou comportant une partie fémorale suivant l'invention.

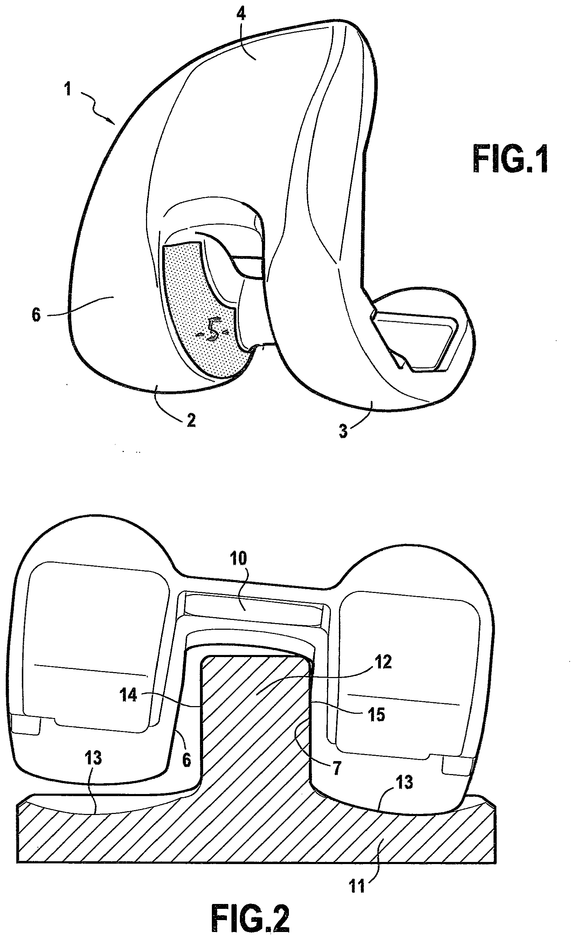

La partie fémorale 1, notamment métallique, comporte deux condyles 2 et 3, gauche et droit, terminés par une partie 4 dite de trochlée. Entre les deux condyles 2 et 3 s'étend un espace intercondyle, également appelé cage 5 de postéro stabilisation. Chaque condyle comporte un flanc respectif gauche et droit 6 et 7, qui se font face mutuellement et délimitent latéralement la cage 5. D'autre part, une came 10 fémorale s'étend d'un condyle à l'autre dans la direction médiolatérale (la direction horizontale à la figure 2).

La prothèse totale de genou comporte en outre une partie tibiale comportant un plateau sur lequel est disposé un ménisque 11 en polyéthylène. Le ménisque 11 comporte sur sa face supérieure, sur laquelle viennent glisser ou rouler les condyles 2 et 3 lors de la flexion relative de la partie fémorale par rapport à la partie tibiale, deux surfaces 13 concaves de forme sensiblement complémentaire des surfaces extérieures des condyles 2 et 3.

En outre, le ménisque 11 comporte un plot formant contre came 12 s'étendant dans la direction verticale à partir de la base du ménisque.

Ce plot 12 formant contre came s'étend, pendant toute la flexion, (flexion de 0 et 130° du genou), à l'intérieur de la cage 5. Lors de la rotation relative de la partie 1 fémorale par rapport au ménisque 11, la came fémorale 10 vient en contact avec une face postérieure du plot 12.

Le plot 12 comporte deux faces latérales gauche et droite 14 et 15 planes verticales, notamment parallèles à l'axe longitudinal de la prothèse et mutuellement opposées, notamment parallèles. La face 14 latérale gauche fait face au flanc 6 gauche, tandis que la face 15 latérale droite fait face au flanc 7 droit.

Lorsqu'un basculement a lieu, notamment par soulèvement latéral de l'implant, notamment du condyle 6 lors de la flexion (effet lift-off comme représenté à la figure 2), la face 15 latérale droite du plot 12 vient en contact surface contre surface avec le flanc 7.

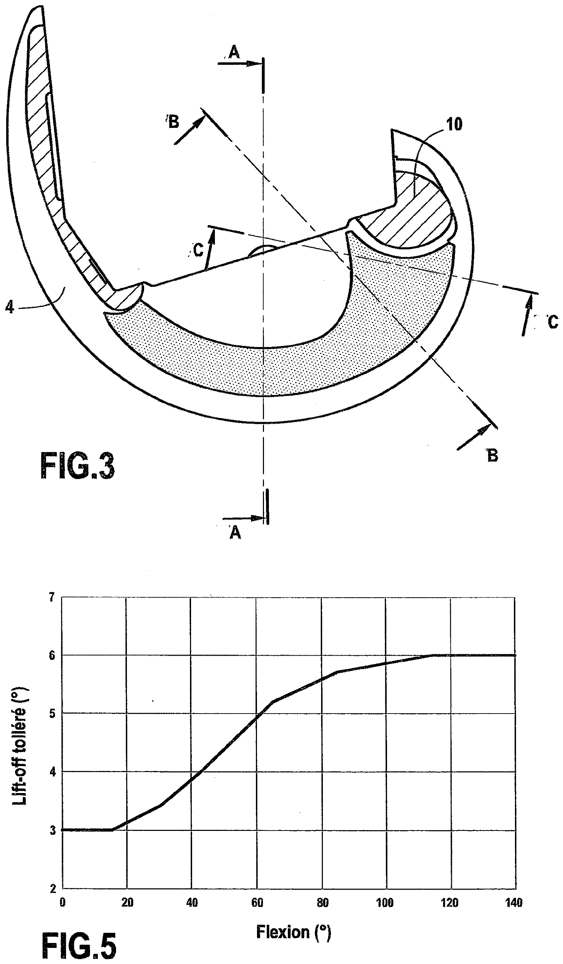

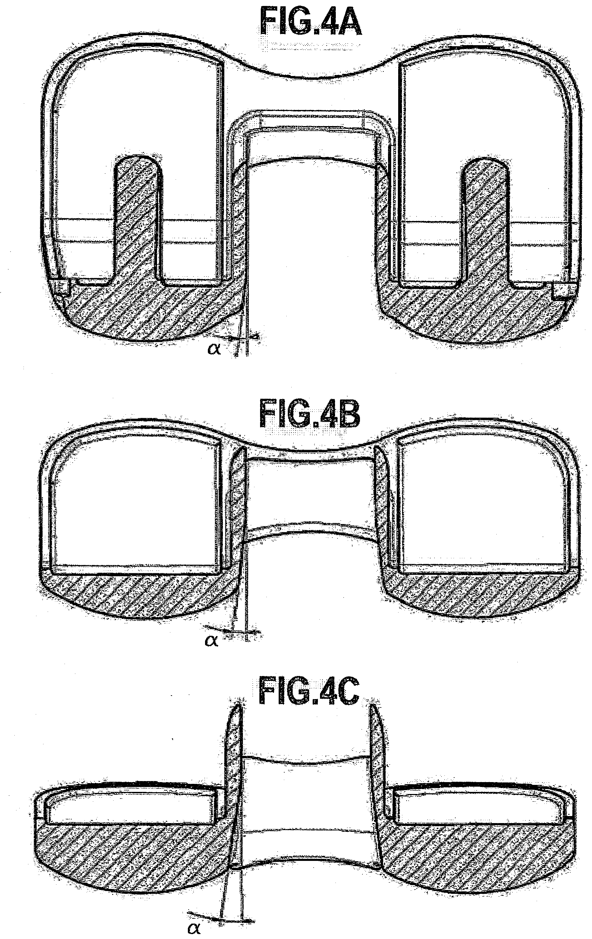

Comme on le voit aux figures 4A, 4B et 4C, l'angle α d'inclinaison augmente au fur à mesure que l'on se déplace de la partie 4 de trochlée vers la came 10 fémorale, c'est à dire au fur à mesure que la flexion augmente, notamment entre 18° et 110°. La courbe donnant la variation de l'angle d'inclinaison en fonction de la flexion pour la prothèse de la figure 3 est donnée à la figure 5. Bien que cette courbe soit favorable en terme d'efficacité anti effet lift off, on pourrait cependant prévoir une autre forme de cette courbe, notamment linéaire.

Dans le mode de réalisation décrit ci dessus, les deux faces opposées sont planes et parallèles, notamment verticales. Cependant, aucune de ces caractéristiques n'est essentielle à l'invention. En particulier les deux faces ne sont pas nécessairement planes. Par exemple, les deux faces peuvent être incurvées, le plot ayant par exemple une section transversale (c'est à dire parallèlement au plan du plateau tibial) qui est circulaire, chaque face respectivement gauche et droit ayant, en section transversale la forme d'un demi cercle, les deux demi cercles gauche et droit étant en opposition.

## Revendications

1. Une prothèse totale de genou constituée de la partie fémorale comportant deux condyles gauche et droit entre lesquels est définie une cage de postéro stabilisation définie par deux flancs gauche et droit opposés dans la direction sagittale, d'un ménisque et d'une partie tibiale comportant un plateau tibial, sur lequel se trouve le ménisque, notamment en un matériau moins dur que le matériau en lequel est réalisée la partie fémorale, le ménisque comportant un plot (12) faisant saillie verticalement du plateau tibial pour être reçu au moins en partie dans la cage entre les deux flancs opposés, **caractérisée en ce que** le plot (12) du ménisque a deux faces opposées gauche et droite verticales faisant face mutuellement aux flancs gauche et droit respectifs de la cage et en section dans le plan perpendiculaire à la direction antéropostérieure, dans au moins une position de flexion du genou comprise entre 0 et 135°, notamment à 60° de flexion, au moins l'un des flancs gauche et droit de la cage est incliné en s'évasant vers l'extérieur respectivement par rapport à au moins l'une des faces gauche et droite du plot, notamment d'un angle d'environ 5° et **en ce que** l'angle d'inclinaison augmente au fur à mesure que la flexion augmente, notamment entre 18° et 110°.

2. Prothèse totale de genou suivant la revendication 1, **caractérisée en ce que** les flancs gauche et droit de la cage sont inclinés respectivement par rapport aux faces gauche et droite du plot, notamment d'un angle d'environ 5°.

3. Prothèse totale de genou suivant l'une des revendications précédentes, **caractérisée en ce que**, les faces gauche et droite opposées du plot sont planes, de préférence mutuellement parallèles.

4. Prothèse totale de genou suivant l'une des revendications précédentes, **caractérisée en ce que** la forme des flancs de la cage est réalisé de sorte que, pour tout angle de flexion de la prothèse, notamment compris entre 0 et 135°, le flanc est incliné par rapport aux faces latérales opposées du plot de l'insert.

5. Prothèse totale de genou suivant l'une des revendications précédentes, **caractérisée en ce que** le ménisque est en un matériau plus tendre que le matériau en lequel est réalisée la partie fémorale, notamment le ou les condyles.

6. Prothèse totale de genou suivant l'une des revendications précédentes, **caractérisée en ce que** l'angle d'inclinaison augmente au fur et à mesure que la flexion augmente de 18° à 110°.

## Patentansprüche

1. Knie-Vollprothese, bestehend aus dem femoralen Teil, umfassend einen linken und einen rechten Gelenkkopf, zwischen denen ein posteriorer Stabilisierungskäfig ausgebildet ist, der aus zwei linken und rechten Seitenteilen gebildet ist, die einander in der sagittalen Richtung gegenüberstehen, aus einem Meniskus und aus einem tibialen Teil, umfassend eine tibiale Platte, auf der sich der Meniskus befindet, insbesondere aus einem weniger harten Werkstoff als der Werkstoff, aus dem der femorale Teil besteht, wobei der Meniskus einen Block (12) umfasst, der vertikal aus der tibialen Platte ragt, um zumindest teilweise in dem Käfig zwischen den beiden einander gegenüberstehenden Seitenteilen aufgenommen zu werden, **dadurch gekennzeichnet, dass** der Block (12) des Meniskus zwei einander gegenüberliegende vertikale linke und rechte Flächen aufweist, die jeweils dem linken bzw. dem rechten Seitenteil des Käfigs und im Schnitt in der Ebene senkrecht zur antero-posterioren Richtung gegenüberstehen, in mindestens einer Flexionsposition des Knies zwischen 0 und 135°, insbesondere bei 60° Flexion, mindestens eines der linken und rechten Seitenteile des Käfigs geneigt ist, indem es sich nach außen bezüglich jeweils mindestens einer der linken und der rechten Fläche des Blocks aufweitet, insbesondere in einem Winkel von ungefähr 5°, und dass sich der Neigungswinkel in dem Maße vergrößert, wie sich die Flexion vergrößert, insbesondere zwischen 18° und 110°.

2. Knie-Vollprothese nach Anspruch 1, **dadurch gekennzeichnet, dass** das linke und das rechte Seitenteil des Käfigs jeweils bezüglich der linken und der rechten Fläche des Blocks geneigt sind, insbesondere in einem Winkel von ungefähr 5°.

3. Knie-Vollprothese nach einem der vorhergehenden Ansprüche, **dadurch gekennzeichnet, dass** die gegenüberstehenden linken und rechten Flächen des Blocks eben sind und vorzugsweise zueinander parallel stehen.

4. Knie-Vollprothese nach einem der vorhergehenden Ansprüche, **dadurch gekennzeichnet, dass** die Form der Seitenteile des Käfigs derart ausgebildet ist, dass bei jedem Flexionswinkel der Prothese, insbesondere zwischen 0 und 135°, das Seitenteil bezüglich den gegenüberliegenden Seitenflächen des Blocks des Einsatzes geneigt ist.

5. Knie-Vollprothese nach einem der vorhergehenden Ansprüche, **dadurch gekennzeichnet, dass** der Meniskus aus einem weicheren Werkstoff besteht als der Werkstoff, aus dem der femorale Teil besteht, insbesondere der Gelenkkopf oder die Gelenkköpfe.

6. Knie-Vollprothese nach einem der vorhergehenden Ansprüche, **dadurch gekennzeichnet, dass** sich der Neigungswinkel in dem Maße vergrößert, wie sich die Flexion von 18° auf 110° vergrößert.

## Claims

1. A total knee prosthesis consisting of a femoral component comprising two left and right condyles between which is a posterior stabilisation cage defined by two left and right sides opposed in the sagittal direction, a meniscus and a tibial component forming a tibial plateau, on which the meniscus is located, made from a softer material than that from which the femoral component is made, the meniscus comprises a post (12) projecting vertically from the tibial plateau positioned, at least in part, within the cage between the two opposite sides, **characterised in that** the post (12) of the meniscus has two opposing left and right vertical edges facing towards the left and right sides of the cage respectively and in cross section in the plane perpendicular to the antero-posterior direction, in at least one flexion position of the knee between 0 and 135°, in particular at 60° flexion, at least one of the left and right sides of the cage is inclined outwards in relation to at least one of the left and right sides of the post respectively, in particular at an angle of about 5° in which the angle of inclination increases as the flexion increases, in particular between 18° and 110°.

2. A total knee prosthesis according to claim 1, **characterised in that** the left and right sides of the cage are inclined in relation to the left and right sides of the post respectively, in particular at an angle of about 5°.

3. A total knee prosthesis according to one of the preceding claims, **characterised in that** the opposite left and right sides of the post are flat and preferably parallel to each other.

4. A total knee prosthesis according to one of the preceding claims, **characterised in that** the shape of the sides of the cage is such that, for any angle of flexion of the prosthesis, in particular between 0 and 135°, the side is inclined in relation to the opposing lateral sides of the post of the insert.

5. A total knee prosthesis according to one of the preceding claims, **characterised in that** the meniscus is made from a softer material than that from which the femoral component is made, in particular the condyle(s).

6. A total knee prosthesis according to one of the preceding claims, **characterised in that** the angle of inclination increases as the flexion increases from 18° to 110°.
